# EUROPEAN PATENT APPLICATION

(11) **EP 3 960 034 A1**
(43) Date of publication of application: **02.03.2022**
(21) Application number: 20815624.0
(22) Date of filing: 20.05.2020
(51) Int. Cl.: A47C 3/00, A47C 7/00, A47C 7/62, A61F 5/01

(54) **SPINE-PROTECTING AND SPINE-CORRECTING MULTIFUNCTIONAL HEALTHCARE SEAT DEVICE EMPLOYING BODY WEIGHT TO ACHIEVE EMBRACE-LIKE SUPPORT, PRESSURE-REDUCTION, FIXING, AND CORRECTION**

(30) Priority: 31.05.2019 CN 201920817538 U; 31.05.2019 CN 201910467356
(71) Applicant: Ye, Zhifei, Hangzhou, Zhejiang 311700 (CN)
(72) Inventor: YE, Zhifei, Hangzhou, Zhejiang 311700 (CN); YE, Lidu, Hangzhou, Zhejiang 311700 (CN)
(74) Representative: Vesterinen, Jussi Tapio
(86) International application number: PCT/CN2020/091216
(87) International publication number: WO 2020/238718

(57) **Abstract**

A spine-protecting and spine-correcting multi-functional health-care seat device employing gravity to achieve embrace-like support, pressure reduction, fixing, and correction, including a seat frame and a gravity embrace-like support and sitting device. The gravity embrace-like support and sitting device includes a sitting plate (1), sitting plate support rods (8) symmetrically arranged on two sides of a bottom of the sitting plate (1), rotating bearings (1101) symmetrically arranged on two sides of the seat frame, embrace-like support levers cooperatively arranged on the rotating bearings (1101), and embrace-like support palms (2) arranged at top ends of the embrace-like support levers. One end of each sitting plate support rod (8) is hinged to a bottom of the sitting plate (1), and the other end is hinged to a bottom of each embrace-like support lever through a support rod rotating shaft (15). The health-care seat device employs the lever principle and the torque balance principle, enables a sitting action and an embrace-like support action to appear and disappear at the same time by means of flexible mechanical connection, and effectively and conveniently converts the gravity of a user into an embrace-like support force to the waist and back without an external force or a manual operation.

## Description

### TECHNICAL FIELD

The present disclosure relates to the technical field of health-care equipment, specifically to a spine-protecting and spine-correcting multi-functional health-care seat device employing gravity to achieve embrace-like support, pressure reduction, fixing, and correction.

### BACKGROUND

I. Among walking, standing, sitting, lying, "sitting" is one of the most common basic behaviors in human activities. The ancient doctors said: sitting for a long time is not good for health. The modern medicine has already proved that sitting for a long time is a serious harm to people's health! It is especially harmful for various organs such as the spine and the lower abdomen. However, with the development of the times and the advancement of science and technology, the proportion of manual labor for human work has been greatly reduced. Particularly in the current IT era, as the Internet and the upcoming Industry 4.0 develop, people do their work while seated. In this era, "sitting" is no longer taking a rest of the agrarian age and the early stage of the industrial society, but a necessary "compulsory" work! It is an excessive and unavoidable behavior of most people. Therefore, how to avoid the harm to the body caused by "sitting" is the problem to be solved by this invention.

II. According to the current social situation, the harms caused by sitting for a long time are as follows:
1. Gravity compression: A part that is closer to a support point (the ischium of the hip) of a seat to the body weight is subjected to greater compression of the body weight gravity. The body weight gravity will exert a excessive pressure on weak parts such as the lumbar vertebra, the hip, the pelvis, and the abdominal cavity for a long time, leading to herniated disk, injury to the nerve plexus, painfulness, and failure in living and working normally, and even lower limb paralysis in serious cases. Internal organs of the abdominal cavity and the pelvic cavity are unable to work normally due to the compression and are diseased.
2. Improper posture and curvature of the spine: It is difficult for users to maintain a correct posture in studying and working, and they will unconsciously hunch, laterally bend their spines etc. Especially when people are tired, people's myopia, blockage of neurovascular structures and other blood vessels, body aches and diseases, premature senility, body being out of shape and not straight, adolescent dysplasia, etc. are directly caused. The poor forward-bending sitting posture aggravates the compression on the abdominal cavity and the pelvic cavity, which aggravates lesions of the glands, organs, and internal organs in the cavities.

III. Current situation of existing seat technology:

According to a technical research on the current seat market:
1. Aiming at the harm 1 -gravity compression No seat device has been found to have a technical function to solve this problem.
2. Aiming at the harm 2-improper posture and curvature of the spine. There are many so-called health-care seats having a function of keeping the spine normal. After investigation, it is found that in fact, a little auxiliary effect can be achieved only if people consciously make their spines fitted to the seat. Generally, when people concentrate on working and studying, with their hands doing and eyes seeing, basically no one will consciously and actively lean against that backrest, so little effect is achieved! In addition, health-care massage chairs on the market only have a certain effect on the back in terms of relaxing the muscles and stimulating the blood circulation, cannot solve the above harms, and can only be used during maintenance and rest. It cannot avoid the harms caused by "sitting" during a lot of work and in living and studying periods.

### SUMMARY

For the problems in the existing art, the present disclosure provides a spine-protecting and spine-correcting multi-functional health-care seat device employing gravity to achieve embrace-like support, pressure reduction, fixing, and correction, which has a scientific and reasonable structure and ingenious design.

The technical solution of the present disclosure is as follows:

A spine-protecting and spine-correcting multi-functional health-care seat device employing gravity to achieve embrace-like support, pressure reduction, fixing, and correction includes a seat frame and a gravity embrace-like support and sitting device arranged on the seat frame. The gravity embrace-like support and sitting device converts the gravity of a user into an embrace-like support force to the waist and back of the user, thus realizing an up support, stabilization, and correction for the waist and back of the user, relieving the pressure on the waist and hip parts, and avoiding the spine deformity.

In the spine-protecting and spine-correcting multi-functional health-care seat device employing gravity to achieve embrace-like support, pressure reduction, fixing, and correction, the gravity embrace-like support and sitting device includes a sitting plate, sitting plate support rods symmetrically arranged on two sides of a bottom of the sitting plate, rotating devices symmetrically arranged on two sides of the seat frame, embrace-like support levers cooperatively arranged on the rotating devices, and embrace-like support palms arranged at top ends of the embrace-like support levers. One end of each sitting plate support rod is hinged to a bottom of the sitting plate, and the other end is hinged to a bottom of each embrace-like support lever through a support rod rotating shaft. The sitting plate can drive the sitting plate support rods to move, and the sitting plate support rods can drive the embrace-like support levers to move; and the embrace-like support levers can rotate on the seat frame through the rotating devices, thus realizing movement of the embrace-like support palms.

In the spine-protecting and spine-correcting multi-functional health-care seat device employing gravity to achieve embrace-like support, pressure reduction, fixing, and correction, the seat frame includes a bottom frame and a U-shaped horizontal bracket arranged on the bottom frame; the bottom frame includes a roller base and an L-shaped bracket arranged on the roller base; the L-shaped bracket is provided with an adjustable headrest; the U-shaped horizontal bracket includes a horizontal transverse rod and horizontal longitudinal rods arranged at two ends of the horizontal transverse rod; the L-shaped bracket of the bottom frame is further provided with a U-shaped vertical support frame; the U-shaped vertical support frame includes a transverse rod and vertical rods arranged at two ends of the transverse rod; the vertical rods are supported at end parts of the horizontal longitudinal rods or positions close to the end parts; the rotating devices adopt rotating bearings; the rotating bearings are arranged at the end parts of the horizontal longitudinal rods or close to the end parts; and inner rings of the rotating bearings are in tight fit with the horizontal longitudinal rods.

In the spine-protecting and spine-correcting multi-functional health-care seat device employing gravity to achieve embrace-like support, pressure reduction, fixing, and correction, the seat frame includes a bottom plate and support pillars arranged at four corners of the bottom plate; tops of the support pillars are provided with bearing pedestals; the rotating devices adopt rotating shafts, and two ends of the rotating shafts are respectively fitted in the bearing pedestals; through holes are formed in the rotating shafts; the embrace-like support levers include embrace-like support connecting rods; the embrace-like support connecting rods are arranged in the through holes in a penetrating manner and are fixedly connected with the rotating shafts; the roller base is fixedly arranged at a bottom of the bottom plate; the adjustable headrest is arranged on a side surface of the bottom plate, thus forming a chair structure; or a binding band is arranged at the bottom of the bottom plate, thus forming a portable seat structure that can be matched with an ordinary chair.

In the spine-protecting and spine-correcting multi-functional health-care seat device employing gravity to achieve embrace-like support, pressure reduction, fixing, and correction, upper arms of the embrace-like support levers are connected with two connecting links, and the connecting links and the embrace-like support levers are connected by movable joint locks, and a horizontal adjustable included angle is 45-90 degrees; an embrace-like support palm skeleton and universal movable joints are arranged on back surfaces of the embrace-like support palms; the connecting links are connected with the movable joints; front surfaces of the embrace-like support palms are made of a cotton and linen material with a high elastic sponge cover friction coefficient and are matched with sunken palm type structures with small upper parts and large lower parts.

In the spine-protecting and spine-correcting multi-functional health-care seat device employing gravity to achieve embrace-like support, pressure reduction, fixing, and correction, each embrace-like support lever includes an embrace-like support rod upper arm, an embrace-like support rod lower arm, and a bearing outer sleeve; the embrace-like support rod upper arm and the embrace-like support rod lower arm are welded at opposite positions on an outer wall of the bearing outer sleeve; the bearing outer sleeve is in tight fit with an outer ring of the rotating bearing; end parts of the horizontal longitudinal rods of the U-shaped horizontal bracket are provided with rotating shaft lock brakes; the rotating shaft lock brakes include threaded rods arranged inside the horizontal longitudinal rods, transmission rods oppositely arranged on the horizontal longitudinal rods in a penetrating manner, and brake handles in screwing fit with the threaded rods; front ends of the brake handles are circumferentially provided with second wedge-shaped surfaces; the transmission rods are provided with reset springs; one end of each reset spring is fixed on each transmission rod, and the other end is fixed on an inner wall of each horizontal longitudinal rod; a third wedge-shaped surface is arranged at one end of each transmission rod close to each threaded rod; a brake pad is arranged at the other end of each transmission rod; an outer edge of each bearing outer sleeve is circumferentially provided with a first wedge-shaped surface; and the brake handles can move on the threaded rods through threaded fit and cooperate with the third wedge-shaped surfaces on the transmission rods through the second wedge-shaped surfaces, thus driving the transmission rods to do relative motion, so that the brake pads are fitted to the first wedge-shaped surfaces to perform a brake action.

In the spine-protecting and spine-correcting multi-functional health-care seat device employing gravity to achieve embrace-like support, pressure reduction, fixing, and correction, each embrace-like support lever includes an embrace-like support rod and a bearing outer sleeve; the bearing outer sleeve is in tight fit with an outer ring of each rotating bearing; the embrace-like support rod is provided with one group of hooks; a ringlike groove is formed in the bearing outer sleeve; the embrace-like support rod is clamped and fixed in the ringlike groove of the bearing outer sleeve through the hooks; a lower end of the embrace-like support rod is provided with a tension spring; one end of the tension spring is fixedly connected with the embrace-like support rod, and the other end is provided with a spring force adjustment device; the spring force adjustment device includes a shell, an upper rack, a lower rack, and a gear; the gear is engaged with the upper rack and the lower rack; the upper rack and the lower rack are arranged in the shell in a sliding fit manner; the upper rack and the lower rack are fixedly connected with the tension spring, respectively; the gear is connected with a rotatable adjustment handle; a rotating shaft of the rotatable adjustment handle is provided with a lock cylinder; a lock hole is formed in a position, corresponding to the lock cylinder, on the shell, and the lock cylinder can cooperate with the lock hole for locking; a front end of the rotating shaft of the rotatable adjustment handle is provided with a spring used to axially reset the rotatable adjustment handle; one end of the spring is fixedly connected with the rotating shaft of the rotatable adjustment handle, and the other end is fixedly connected with an inner wall of the shell.

In the spine-protecting and spine-correcting multi-functional health-care seat device employing gravity to achieve embrace-like support, pressure reduction, fixing, and correction, tail ends of the rotating shafts are provided with rotating shaft lock brakes; the rotating shaft lock brakes include threaded rods arranged inside the rotating shafts, transmission rods oppositely arranged on the rotating shafts in a penetrating manner, and brake handles in screwing fit with the threaded rods; front ends of the brake handles are circumferentially provided with second wedge-shaped surfaces; the transmission rods are provided with reset springs; one end of each reset spring is fixed on each transmission rod, and the other end is fixed on an inner wall of each rotating shaft; a third wedge-shaped surface is arranged at one end of each transmission rod close to each threaded rod; a brake pad is arranged at the other end of each transmission rod; an outer edge of each bearing pedestal is circumferentially provided with a first wedge-shaped surface; and the brake handles can move on the threaded rods through threaded fit and cooperate with the third wedge-shaped surfaces on the transmission rods through the second wedge-shaped surfaces, thus driving the transmission rods to do relative motion, so that the brake pads are fitted to the first wedge-shaped surfaces to perform a brake action.

In the spine-protecting and spine-correcting multi-functional health-care seat device employing gravity to achieve embrace-like support, pressure reduction, fixing, and correction, pin holes located on side surfaces of the through holes are formed in the rotating shafts, and the pin holes communicate with the through holes; one group of spring pins are arranged on the embrace-like support connecting rods; the embrace-like support connecting rods are arranged on the rotating shafts through the through holes in a penetrating manner and cooperate with the pin holes through spring pins to realize upper-lower ratio adjustment of the embrace-like support connecting rods on the rotating shafts; a lower end of each embrace-like support rod is provided with a tension spring; one end of the tension spring is fixedly connected with the embrace-like support connecting rod, and the other end is provided with a spring force adjustment device; the spring force adjustment device includes a shell, an upper rack, a lower rack, and a gear; the gear is engaged with the upper rack and the lower rack; the upper rack and the lower rack are arranged in the shell in a sliding fit manner; the upper rack and the lower rack are fixedly connected with the tension spring, respectively; the gear is connected with a rotatable adjustment handle; a rotating shaft of the rotatable adjustment handle is provided with a lock cylinder; a lock hole is formed in a position, corresponding to the lock cylinder, on the shell, and the lock cylinder can cooperate with the lock hole for locking; a front end of the rotating shaft of the rotatable adjustment handle is provided with a spring used to axially reset the rotatable adjustment handle; one end of the spring is fixedly connected with the rotating shaft of the rotatable adjustment handle, and the other end is fixedly connected with an inner wall of the shell.

In the spine-protecting and spine-correcting multi-functional health-care seat device employing gravity to achieve embrace-like support, pressure reduction, fixing, and correction, tension springs are arranged in gaps between the embrace-like support levers and the sitting plate support rods; one end of each tension spring is fixedly connected with each embrace-like support lever, and the other end is fixedly connected with the sitting plate support rod.

The present disclosure has the beneficial effects:
1) By means of the arrangement of the gravity embrace-like support and sitting device, on the basis of the lever principle and the moment balance principle, through the mechanical flexible connection, the pressure applied by the gravity of the user on the ischium at the bottom of the spine can be effectively transformed into an embrace-like support force to the waist and back by the linkage generated among the sitting plate, the sitting plate support rods, the embrace-like support levers, and the embrace-like support palms; the two kinds of forces mutually reinforce and neutralize each other and are automatically dynamically balanced under a total body weight force; and they respectively bear the body weight together at their positions, which greatly reduces the compression of the gravity on the waist, the hip, and the abdominopelvic cavity! At the same time, the spine can be straightened and stabilized without deformation. The way and connotation that the sitting behavior of humans for thousands of years all relies on the hip are revolutionized, various harms caused by the original unavoidable "sitting" behavior of humans to the health are avoided and prevented, and effects of protecting the spine and correcting the spine are achieved.
2) The seat frame is provided with the rotating shaft lock brakes, which can effectively control a rotation magnitude of the embrace-like support levers to adjust the embrace-like support forces of the embrace-like support palms. At the same time, the rotating shaft lock brake can also control the speeds of the embrace-like support levers, and the gravity is allowed to remain on the sitting plate to a certain extent, so as to adapt to sitting requirements of different persons.
3) There are four embrace-like support palms on the seat frame, which can realize multi-point separate support for the waist and back of the user. At the same time, each embrace-like support palm adopts the sunken palm structure with a small upper part and a large lower part, so as to improve the fitness between the waist and back of the user, which makes the embrace-like support action softer, stabler, and more comfortable.
4) Each embrace-like support lever adopts an adjustable structure, which can adjust a ratio of the upper arm to the lower arm of the embrace-like support lever, and the embrace-like support lever is assisted by the adjustable tension spring, so that the pressures of the embrace-like support palms on the waist and back of the user can be effectively adjusted, and adjustment of various experiences is realized, so as to better adapt to different users.
5) No energy is consumed. The gravity (the earth gravity) is converted into the embrace-like support force, without a need of external energy for supporting. This is called Unarmed Counter Move.
6) It is extremely convenient. After the mode is set according to personal details, the embrace-like support action and the "sitting" behavior will appear and disappear at the same time. There is no need to add more operating procedures than the previous seat.
7) Along the gravity direction, the spine is more natural and upright.
8) In addition to avoiding and preventing the harms of sitting, the present disclosure can also play an adjuvant therapy role for diseases that have been caused by traditional "sitting" work, without stopping the work that is done while the user is seated.
9) The present disclosure has multiple effects, is extremely convenient to use and comfortable, and has unique, scientific, ingenious concept and design, and the seat device is revolutionary.

### BRIEF DESCRIPTION OF THE DRAWINGS

FIG. 1 is a schematic diagram of an overall three-dimensional structure of Embodiment 1 of the present disclosure;
FIG. 2 is a schematic diagram of an overall three-dimensional structure of Embodiment 2 of the present disclosure;
FIG. 3 is a schematic diagram of an overall three-dimensional structure of Embodiment 3 of the present disclosure;
FIG. 4 is a schematic structural diagram of a portable seat of Embodiment 3 of the present disclosure;
FIG. 5 is a schematic structural diagram of a chair of Embodiment 3 of the present disclosure;
FIG. 6 is a schematic diagram of an overall three-dimensional structure of Embodiment 4 of the present disclosure;
FIG. 7 is a schematic structural diagram of a portable seat of Embodiment 4 of the present disclosure;
FIG. 8 is a schematic structural diagram of a chair of Embodiment 4 of the present disclosure;
FIG. 9 is an enlarged schematic diagram of a structure at Part A of the present disclosure;
FIG. 10 is a schematic diagram of an internal structure of a rotating shaft lock brake of Embodiment 1 of the present disclosure;
FIG. 11 is a schematic diagram of a three-dimensional structure of a rotating shaft lock brake of Embodiment 3 of the present disclosure;
FIG. 12 is a schematic diagram of an internal structure of a rotating shaft lock brake of Embodiment 3;
FIG. 13 is an enlarged schematic diagram of a structure at Part B of the present disclosure;
FIG. 14 is a schematic diagram of an internal front structure of a spring force adjustment device of the present disclosure;
FIG. 15 is a schematic structural diagram of a back surface of an embrace-like support palm of the present disclosure;
FIG. 16 is a schematic structural diagram of a front surface of an embrace-like support palm of the present disclosure;
FIG. 17 is a schematic diagram of a three-dimensional structure of a spring force adjustment device of the present disclosure; and
FIG. 18 is a schematic diagram of an internal top structure of a spring force adjustment device of the present disclosure.

In the drawings: 1: sitting plate; 2: embrace-like support palm; 201: embrace-like support palm skeleton; 202: universal movable joint; 3: connecting link; 4: embrace-like support rod upper arm; 5: rotating shaft lock brake; 501: brake handle; 502: second wedge-shaped surface; 503: threaded rod; 504: reset spring; 505: transmission rod; 506: brake pad; 507: third wedge-shaped surface; 6: embrace-like support rod lower arm; 7: tension spring; 8: sitting plate support rod; 9: roller base; 10: U-shaped vertical support frame; 11: bearing outer sleeve; 1101: rotating bearing; 1102: ringlike groove; 1103: first wedge-shaped surface; 12: L-shaped bracket; 13: U-shaped horizontal bracket; 14: adjustable headrest; 15: support rod rotating shaft; 16: spring force adjustment device; 1601: shell; 1602: upper rack; 1603: gear; 1604: lower rack; 1605: adjustment handle; 1606: lock hole; 1607: lock cylinder; 1608: spring; 1609: rail; 1610: rail slot; 17: hook; 18: embrace-like support rod; 19: binding band; 20: rotating shaft; 21: embrace-like support connecting rod; 22: spring pin; 23: bearing pedestal; 24: bottom plate; and 25: support pillar.

### DETAILED DESCRIPTION OF THE EMBODIMENTS

The present disclosure is further described below in combination with accompanying drawings of the specification.

### Embodiment 1

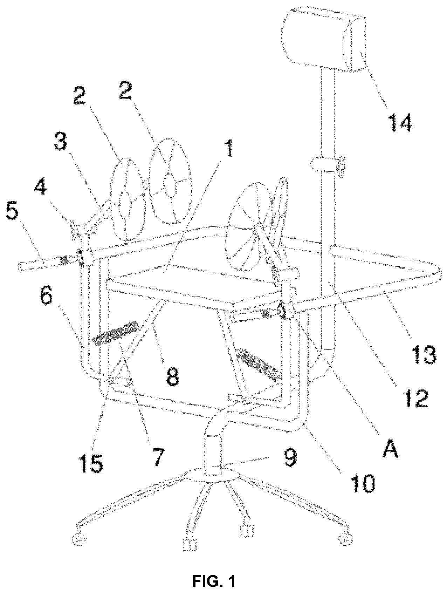

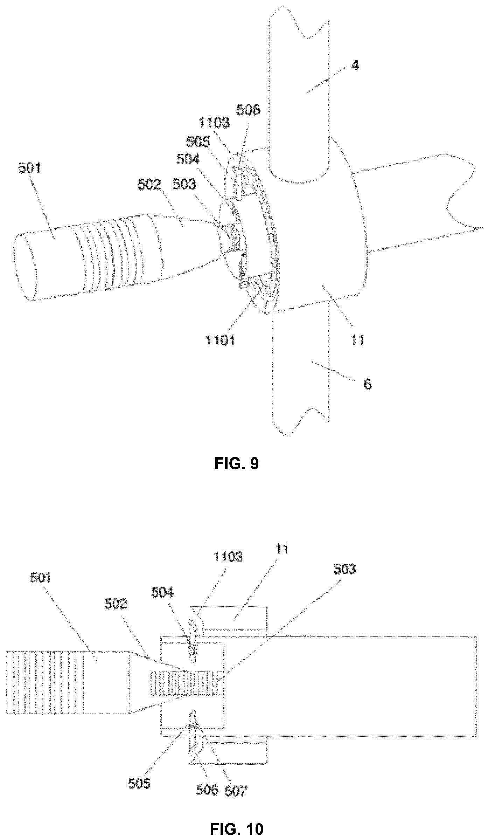

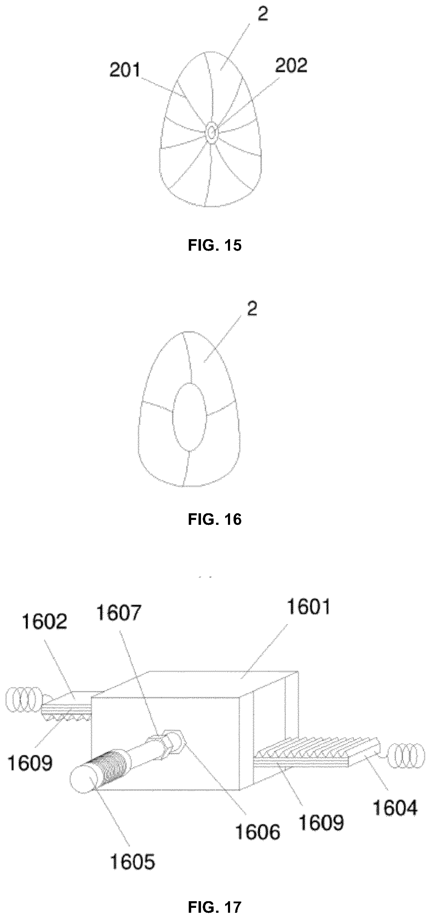

As shown in FIGs. 1, 9, 10, 15, and 16, a spine-protecting and spine-correcting multi-functional health-care seat device employing gravity to achieve embrace-like support, pressure reduction, fixing, and correction includes a seat frame and a gravity embrace-like support and sitting device movably arranged on the seat frame.

The gravity embrace-like support and sitting device includes a sitting plate 1, sitting plate support rods 8 symmetrically arranged on two sides of a bottom of the sitting plate 1, rotating bearings 1101 symmetrically arranged on two sides of the seat frame, embrace-like support levers cooperatively arranged on the rotating bearings 1101, and embrace-like support palms 2 arranged at top ends of the embrace-like support levers. One end of each sitting plate support rod 8 is hinged to a bottom of the sitting plate 1, and the other end is hinged to a bottom of each embrace-like support lever through a support rod rotating shaft 15, thus realizing linkage of the sitting plate 1, the sitting plate support rods 8, the embrace-like support levers, and the embrace-like support palms 2.

The seat frame includes a bottom frame and a U-shaped horizontal bracket 13 arranged on the bottom frame; the bottom frame includes a roller base 9 and an L-shaped bracket 12 arranged on the roller base 9; and the L-shaped bracket 12 is provided with an adjustable headrest 14 (the adjustable headrest 14 may be hinged to the L-shaped bracket 12 for angle adjustment). By the arrangement of the adjustable headrest 14, the head of a user can be well protected, and the sitting comfortability is improved to a certain extent. The U-shaped horizontal bracket 13 includes a horizontal transverse rod and horizontal longitudinal rods arranged at two ends of the horizontal transverse rod. The L-shaped bracket 12 of the bottom frame is further provided with a U-shaped vertical support frame 10; the U-shaped vertical support frame 10 includes a transverse rod and vertical rods arranged at two ends of the transverse rod; and the vertical rods are supported at positions close to end parts of the horizontal longitudinal rods. The U-shaped vertical support frame 10 can effectively support the U-shaped horizontal support frame 13 to improve the overall stability of the seat frame.

The rotating bearings 1101 are arranged at the end parts of the horizontal longitudinal rods; and inner rings of the rotating bearings 1101 are in tight fit, usually interference fit, with the horizontal longitudinal rods.

Each embrace-like support lever includes an embrace-like support rod upper arm 4, an embrace-like support rod lower arm 6, and a bearing outer sleeve 11; the embrace-like support rod upper arm 4 and the embrace-like support rod lower arm 6 are welded at opposite positions on an outer wall of the bearing outer sleeve 11, so that the embrace-like support rod upper arm 4 and the embrace-like support rod lower arm 6 are located on the same line; and the bearing outer sleeve 11 is in tight fit, usually interference fit, with an outer ring of the rotating bearing 1101.

End parts of the horizontal longitudinal rods of the U-shaped horizontal bracket 13 are provided with rotating shaft lock brakes 5; the rotating shaft lock brakes 5 include threaded rods 503 (the threaded rods 503 and the horizontal longitudinal rods are integrated or the threaded rods 503 are fixedly arranged on inner walls of the horizontal longitudinal rods) arranged inside the horizontal longitudinal rods, transmission rods 505 oppositely arranged on the horizontal longitudinal rods in a penetrating manner, and brake handles 501 in screwing fit with the threaded rods 503; front ends of the brake handles 501 are circumferentially provided with second wedge-shaped surfaces 502; the transmission rods 505 are provided with reset springs 504; one end of each reset spring 504 is clamped and fixed on each transmission rod 505, and the other end is welded and fixed on the inner wall of each horizontal longitudinal rod; a third wedge-shaped surface 507 is arranged at one end of each transmission rod 505 close to each threaded rod 503; a brake pad 506 is arranged at the other end of each transmission rod 505; an outer edge of each bearing outer sleeve 11 is circumferentially provided with a first wedge-shaped surface 1103; and the brake handles 501 can move on the threaded rods 503 through threaded fit and cooperate with the third wedge-shaped surfaces 507 on the transmission rods 505 through the second wedge-shaped surfaces 502, thus driving the transmission rods 505 to do relative motion, so that the brake pads 506 are fitted to the first wedge-shaped surfaces 1103 to perform a brake action. The arrangement of the rotating shaft lock brakes 5 can control rotation amplitudes of the embrace-like support levers, thus locking a distribution ratio of the body weight on the sitting plate and embrace-like support palms, or control rotation speeds of the embrace-like support levers to maintain the body weight on the sitting plate in a certain extent, thus adapting to different users. Meanwhile, the brake handles 501 may also be used as armrests to improve the sitting comfortability.

Tension springs 7 are arranged in gaps between the embrace-like support levers and the sitting plate support rods 8; one end of each tension spring 7 is fixedly connected with each embrace-like support lever, and the other end is fixedly connected with the sitting plate support rod 8. The arrangement of the tension springs 7 can reset the gravity embrace-like support and sitting device. This is convenient for the user to sit at the next time.

The embrace-like support levers are provided with two connecting links 3, and the connecting links 3 and the embrace-like support levers are connected by movable joint locks, and a horizontal included angle is preferably 70 degrees; an embrace-like support palm skeleton 201 and universal movable joints 202 are arranged on back surfaces of the embrace-like support palms 2; the connecting links 3 are connected with the movable joints 202, so that upper, lower, left, and right angles of the embrace-like support palms 2 can be automatically adjusted according to the body type; front surfaces of the embrace-like support palms 2 are made of a cotton and linen material with a high elastic sponge cover friction coefficient (i.e., the surface is made of the cotton and linen material and the interior is filled with elastic sponge) and are matched with sunken palm type structures with small upper parts and large lower parts, so that the embrace-like support palms can be more in line with the waist and back of the user, and the embrace-like support action is softer, stabler, and more comfortable.

### Embodiment 2

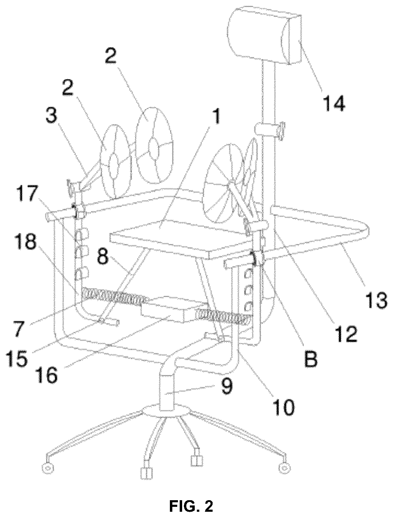

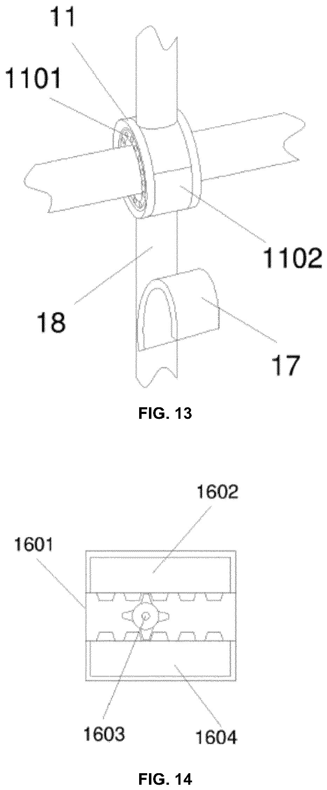

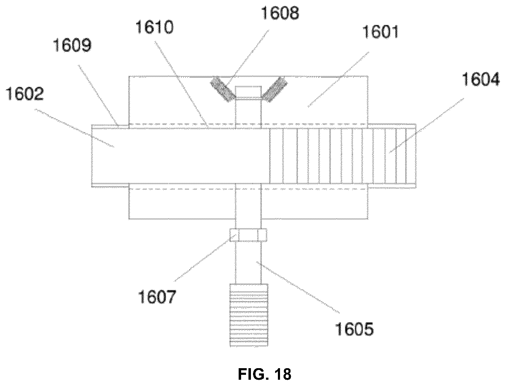

As shown in FIGs. 2, 13, 14, 15, 16, 17, and 18, a spine-protecting and spine-correcting multi-functional health-care seat device employing gravity to achieve embrace-like support, pressure reduction, fixing, and correction includes a seat frame and a gravity embrace-like support and sitting device movably arranged on the seat frame.

The gravity embrace-like support and sitting device includes a sitting plate 1, sitting plate support rods 8 symmetrically arranged on two sides of a bottom of the sitting plate 1, rotating bearings 1101 symmetrically arranged on two sides of the seat frame, embrace-like support levers cooperatively arranged on the rotating bearings 1101, and embrace-like support palms 2 arranged at top ends of the embrace-like support levers. One end of each sitting plate support rod 8 is hinged to a bottom of the sitting plate 1, and the other end is hinged to a bottom of each embrace-like support lever through a support rod rotating shaft 15.

The seat frame includes a bottom frame and a U-shaped horizontal bracket 13 arranged on the bottom frame; the bottom frame includes a roller base 9 and an L-shaped bracket 12 arranged on the roller base 9; and the L-shaped bracket 12 is provided with an adjustable headrest 14. The U-shaped horizontal bracket 13 includes a horizontal transverse rod and horizontal longitudinal rods arranged at two ends of the horizontal transverse rod. The L-shaped bracket 12 of the bottom frame is further provided with a U-shaped vertical support frame 10; the U-shaped vertical support frame 10 includes a transverse rod and vertical rods arranged at two ends of the transverse rod; and the vertical rods are supported at end parts or positions close to the end parts of the horizontal longitudinal rods.

The rotating bearings 1101 are arranged at the end parts of the horizontal longitudinal rods; and inner rings of the rotating bearings 1101 are in tight fit, usually interference fit, with the horizontal longitudinal rods.

Each embrace-like support lever includes an embrace-like support rod 18 and a bearing outer sleeve 11; the bearing outer sleeve11 is in tight fit with an outer ring of each rotating bearing 1101; the embrace-like support rod 18 is provided with one group of hooks 17; a ringlike groove 1102 is formed in the bearing outer sleeve 11; and the embrace-like support rod 18 is clamped and fixed in the ringlike groove 1102 of the bearing outer sleeve 11 through the hooks 17, thus realizing upper-lower ratio adjustment for the embrace-like support rods 18 on the horizontal longitudinal rods and realizing adjustment the body weights separately supported by the sitting plate 1 and the embrace-like support palms 2. For example, if the embrace-like support rods are adjusted to be short in the upper part and long in the lower part, pressures from the embrace-like support palms to the waist and back increase, and a friction force increases, so a total value of the body weight of the user is distributed more to the embrace-like support palms.

A lower end of each embrace-like support rod is provided with a tension spring 7; one end of the tension spring 7 is fixedly connected with the embrace-like support rod, and the other end is provided with a spring force adjustment device 16; the spring force adjustment device 16 includes a shell 1601, an upper rack 1602, a lower rack 1604, and a gear 1603; the gear 1603 is engaged with the upper rack 1602 and the lower rack 1604; the upper rack 1602 and the lower rack 1604 are arranged in the shell 1601 in a sliding fit manner (the upper rack 1602 and the lower rack 1604 are respectively provided with rails 1609; rail slots 1610 are arranged at positions, corresponding to the rails 1609, in the shell 1601; the rails 1609 are cooperatively arranged in the rail slots 1610); the upper rack 1602 and the lower rack 1604 are fixedly connected with the tension spring 7, respectively. The gear 1603 is adjusted to enable the upper rack 1602 and the lower rack 1604 to do a relative motion, thus realizing adjustment of the tension springs 7 on both sides and adjusting rotation angles of the embrace-like support rods 18. The gear 1603 is connected with a rotatable adjustment handle 1605, which is convenient for adjusting the gear; a rotating shaft of the rotatable adjustment handle 1605 is provided with a lock cylinder 1607; a lock hole 1606 is formed in a position, corresponding to the lock cylinder 1607, on the shell 1601, and the lock cylinder 1607 can cooperate with the lock hole 1606 for locking; a front section of the rotating shaft of the rotatable adjustment handle 1605 is provided with a spring 1608 used to axially reset the rotatable adjustment handle 1605; one end of the spring 1608 is fixedly connected with the rotating shaft of the rotatable adjustment handle 1605, and the other end is fixedly connected with an inner wall of the shell 1601.

The rotatable adjustment handle 1605 is pulled out by a distance during adjustment; after the adjustment is completed, under the action of the spring 1608, the rotatable adjustment handle automatically retracts; and the lock cylinder 1607 and the lock hole 1606 cooperate with each other for locking. When the user is seated, the body weight gravity will be automatically distributed on the sitting plate 1 and the embrace-like support palms 2, while the spring force adjustment devices 16 are used to control the body weight to remain on the sitting plate 1, so as to distribute and adjust the gravity together with the hooks 17 on the embrace-like support rods 18.

The embrace-like support levers are provided with two connecting links 3, and the connecting links 3 are connected with the embrace-like support levers through movable joint locks, and a horizontal included angle is preferably 70. An embrace-like support palm skeleton 201 and universal movable joints 202 are arranged on back surfaces of the embrace-like support palms 2; the connecting links 3 are connected with the movable joints 202, so that upper, lower, left, and right angles of the embrace-like support palms 2 can be automatically adjusted according to the body type; front surfaces of the embrace-like support palms 2 are made of a cotton and linen material with a high elastic sponge cover friction coefficient and are matched with sunken palm type structures with small upper parts and large lower parts, so that the embrace-like support palms can be more in line with the waist and back of the user, and the embrace-like support action is softer, stabler, and more comfortable.

### Embodiment 3

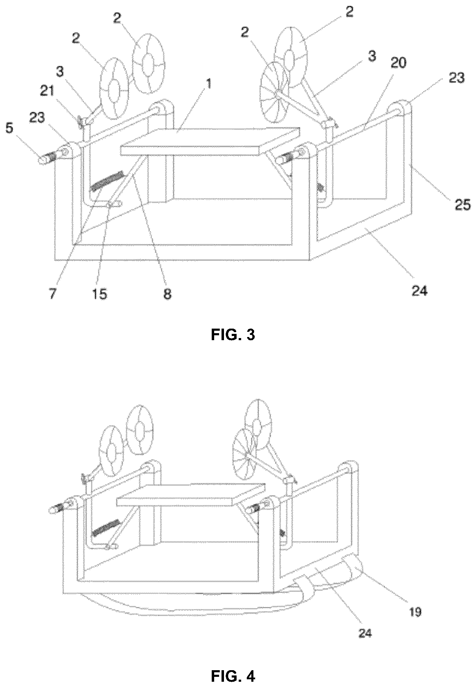

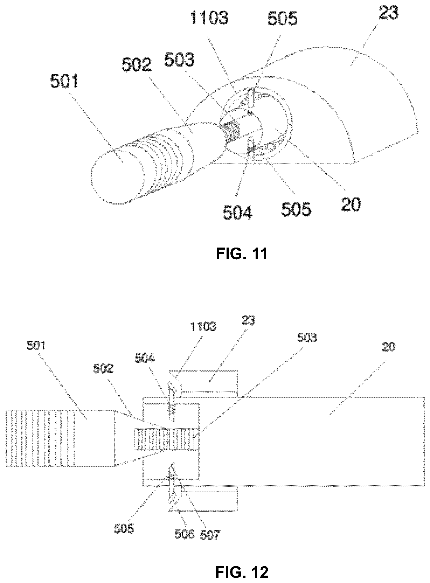

As shown in FIGs. 3, 4, 5, 11, 12, 15, and 16, a spine-protecting and spine-correcting multi-functional health-care seat device employing gravity to achieve embrace-like support, pressure reduction, fixing, and correction includes a seat frame and a gravity embrace-like support and sitting device movably arranged on the seat frame.

The gravity embrace-like support and sitting device includes a sitting plate 1, sitting plate support rods 8 symmetrically arranged on two sides of a bottom of the sitting plate 1, rotating devices symmetrically arranged on two sides of the seat frame, embrace-like support levers cooperatively arranged on the rotating devices, and embrace-like support palms 2 arranged at top ends of the embrace-like support levers. One end of each sitting plate support rod 8 is hinged to a bottom of the sitting plate 1, and the other end is hinged to a bottom of each embrace-like support lever through a support rod rotating shaft 15. The sitting plate 1 can drive the sitting plate support rods 8 to move, and the sitting plate support rods 8 can drive the embrace-like support levers to move; and the embrace-like support levers can rotate on the seat frame through the rotating devices, thus realizing movement of the embrace-like support palms 2.

The seat frame includes a bottom plate 24 and support pillars 25 arranged at four corners of the bottom plate 24; tops of the support pillars 25 are provided with bearing pedestals 23; the rotating devices adopt rotating shafts 20, and two ends of the rotating shafts 20 are respectively fitted in the bearing pedestals 23; through holes are formed in the rotating shafts 20; the embrace-like support levers include embrace-like support connecting rods 21; and the embrace-like support connecting rods 21 are arranged in the through holes in a penetrating manner and are fixedly connected with the rotating shafts 20.

Tension springs 7 are arranged in gaps between the embrace-like support levers and the sitting plate support rods 8; one end of each tension spring 7 is fixedly connected with each embrace-like support lever, and the other end is fixedly connected with the sitting plate support rod 8, so as to automatically reset the gravity embrace-like support and sitting device.

The embrace-like support lever upper arms are provided with two connecting links 3, and the connecting links 3 and the embrace-like support levers are connected by movable joint locks, and a horizontal included angle is preferably 70 degrees; an embrace-like support palm skeleton 201 and universal movable joints 202 are arranged on back surfaces of the embrace-like support palms 2; the connecting links 3 are connected with the movable joints 202, so that upper, lower, left, and right angles of the embrace-like support palms 2 can be automatically adjusted according to the body type; front surfaces of the embrace-like support palms 2 are made of a cotton and linen material with a high elastic sponge cover friction coefficient and are matched with sunken palm type structures with small upper parts and large lower parts, so that the embrace-like support palms can be more in line with the waist and back of the user, and the embrace-like support action is softer, stabler, and more comfortable.

Tail ends of the rotating shafts 20 are provided with rotating shaft lock brakes 5; the rotating shaft lock brakes 5 include threaded rods 503 arranged inside the rotating shafts 20, transmission rods 505 arranged at opposite positions of the rotating shafts 20 in a penetrating manner, and brake handles 501 in screwing fit with the threaded rods 503; front ends of the brake handles 501 are circumferentially provided with second wedge-shaped surfaces 502; the transmission rods 505 are provided with reset springs 504; one end of each reset spring 504 is fixed on each transmission rod 505, and the other end is fixed on an inner wall of each rotating shaft 20; a third wedge-shaped surface 507 is arranged at one end of each transmission rod 505 close to each threaded rod 503; a brake pad 506 is arranged at the other end of each transmission rod 505; an outer edge of each bearing pedestal 23 is circumferentially provided with a first wedge-shaped surface 1103; and the brake handles 501 can move on the threaded rods 503 through threaded fit and cooperate with the third wedge-shaped surfaces 507 on the transmission rods 505 through the second wedge-shaped surfaces 502, thus driving the transmission rods 505 to do relative motion, so that the brake pads 506 are fitted to the first wedge-shaped surfaces 1103 to perform a brake action. The arrangement of the rotating shaft lock brakes 5 can control rotation amplitudes of the embrace-like support levers, thus locking a distribution ratio of the body weight on the sitting plate and embrace-like support palms, or control rotation speeds of the embrace-like support levers to maintain the body weight on the sitting plate in a certain extent, thus adapting to different users. Meanwhile, the brake handles 501 may also be used as armrests to improve the sitting comfortability.

A roller base 9 is fixedly arranged at a bottom of a bottom plate 24; an adjustable headrest 14 is arranged on a side surface of the bottom plate 24, thus forming a chair structure; or a binding band 19 is arranged at the bottom of the bottom plate 24, thus forming a portable seat structure that can be matched with an ordinary chair.

### Embodiment 4

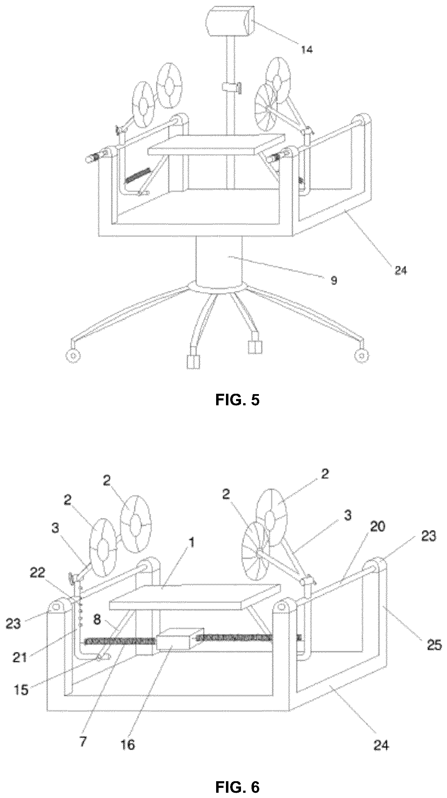

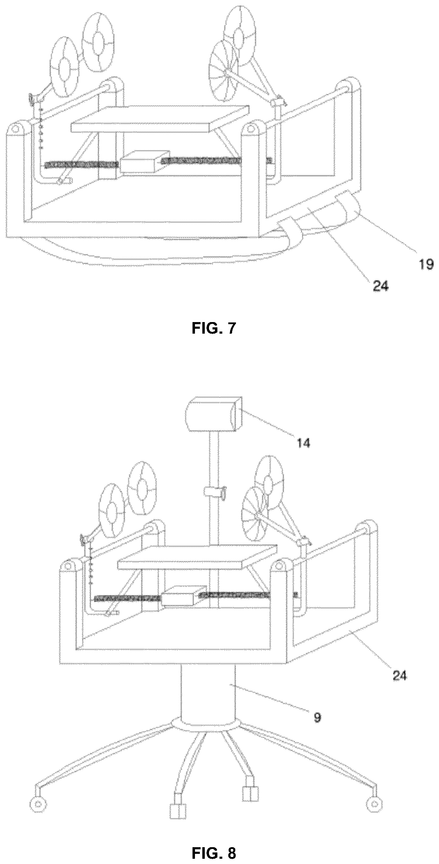

As shown in FIGs. 6, 7, 8, 14, 15, 16, 17, and 18, a spine-protecting and spine-correcting multi-functional health-care seat device employing gravity to achieve embrace-like support, pressure reduction, fixing, and correction includes a seat frame and a gravity embrace-like support and sitting device movably arranged on the seat frame.

The gravity embrace-like support and sitting device includes a sitting plate 1, sitting plate support rods 8 symmetrically arranged on two sides of a bottom of the sitting plate 1, rotating devices symmetrically arranged on two sides of the seat frame, embrace-like support levers cooperatively arranged on the rotating devices, and embrace-like support palms 2 arranged at top ends of the embrace-like support levers. One end of each sitting plate support rod 8 is hinged to a bottom of the sitting plate 1, and the other end is hinged to a bottom of each embrace-like support lever through a support rod rotating shaft 15. The sitting plate 1 can drive the sitting plate support rods 8 to move, and the sitting plate support rods 8 can drive the embrace-like support levers to move; and the embrace-like support levers can rotate on the seat frame through the rotating devices, thus realizing movement of the embrace-like support palms 2.

The seat frame includes a bottom plate 24 and support pillars 25 arranged at four corners of the bottom plate 24; tops of the support pillars 25 are provided with bearing pedestals 23; the rotating devices adopt rotating shafts 20, and two ends of the rotating shafts 20 are respectively fitted in the bearing pedestals 23; through holes are formed in the rotating shafts 20; the embrace-like support levers include embrace-like support connecting rods 21; and the embrace-like support connecting rods 21 are arranged in the through holes in a penetrating manner and are fixedly connected with the rotating shafts 20.

The embrace-like support lever uppers are connected and provided with two connecting links 3, and the connecting links 3 are connected with the embrace-like support levers through movable joint locks, and a horizontal adjustable included angle is 45-90 degrees. An embrace-like support palm skeleton 201 and universal movable joints 202 are arranged on back surfaces of the embrace-like support palms 2; the connecting links 3 are connected with the movable joints 202; front surfaces of the embrace-like support palms 2 are made of a cotton and linen material with a high elastic sponge cover friction coefficient and are matched with sunken palm type structures with small upper parts and large lower parts.

Pin holes located on side surfaces of the through holes are formed in the rotating shafts 20, and the pin holes communicate with the through holes; one group of spring pins 22 are arranged on the embrace-like support connecting rods 21; the embrace-like support connecting rods 21 are arranged on the rotating shafts 20 through the through holes in a penetrating manner and cooperate with the pin holes through spring pins 22 to realize upper-lower ratio adjustment of the embrace-like support connecting rods 21 on the rotating shafts 20; and therefore, adjustment of the body weight separately supported by the sitting plate 1 and the embrace-like support palms 2 is realized. For example, if the embrace-like support connecting rods are adjusted to be long in the upper part and short in the lower part, pressures to the waist and back decrease, and a friction force from the embrace-like support palms to the body decreases, so a total value of the body weight of the user is distributed less to the embrace-like support palms.

A lower end of each embrace-like support connecting rod 21 is provided with a tension spring 7; one end of the tension spring 7 is fixedly connected with the embrace-like support connecting rod 21, and the other end is provided with a spring force adjustment device 16; the spring force adjustment device 16 includes a shell 1601, an upper rack 1602, a lower rack 1604, and a gear 1603; the gear 1603 is engaged with the upper rack 1602 and the lower rack 1604; the upper rack 1602 and the lower rack 1604 are arranged in the shell 1601 in a sliding fit manner; and the upper rack 1602 and the lower rack 1604 are fixedly connected with the tension spring 7, respectively. A rotating shaft of the rotatable adjustment handle 1605 is provided with a lock cylinder 1607; a lock hole 1606 is formed in a position, corresponding to the lock cylinder 1607, on the shell 1601, and the lock cylinder 1607 can cooperate with the lock hole 1606 for locking; a front section of the rotating shaft of the rotatable adjustment handle 1605 is provided with a spring 1608 used to axially reset the rotatable adjustment handle 1605; one end of the spring 1608 is fixedly connected with the rotating shaft of the rotatable adjustment handle 1605, and the other end is fixedly connected with an inner wall of the shell 1601.

The rotatable adjustment handle 1605 is pulled out by a distance during adjustment; after the adjustment is completed, under the action of the spring 1608, the rotatable adjustment handle automatically retracts; and the lock cylinder 1607 and the lock hole 1606 cooperate with each other for locking. When the user is seated, the body weight gravity will be automatically distributed on the sitting plate 1 and the embrace-like support palms 2, while the spring force adjustment devices 16 are used to control the body weight to remain on the sitting plate 1, so as to distribute and adjust the weight together with spring pins 22 and pin holes.

A roller base 9 is fixedly arranged at a bottom of a bottom plate 24; an adjustable headrest 14 is arranged on a side surface of the bottom plate 24, thus forming a chair structure; or a binding band 19 is arranged at the bottom of the bottom plate 24, thus forming a portable seat structure that can be matched with an ordinary chair.

### Working principle:

When a user sits on the sitting plate 1, under the action of the weight of the user, the sitting plate 1 moves down, thereby driving the sitting plate support rods 8 connected with the sitting plate 1 to be opened towards both sides, so that the embrace-like support levers rotate in the seat frame; the bottoms of the embrace-like support levers move outwards, and the tops of the embrace-like support levers move inwards; the embrace-like support palms 2 are fitted to the waist and back of the user through the connecting links 3; the embrace-like support palms 2 generate an upward embrace-like support force to the waist and back of the user, and the waist and back of the user get an upward support, which reduces the pressure applied by the gravity to the sitting plate 1; and the forces born by the sitting plate 1 and the embrace-like support palms 2 mutually reinforce and neutralize each other, so people "embrace and support themselves upright". Under the total value of the body weight gravity and a mode ratio preset by the user, a dynamic balance is automatically obtained, thus effectively reducing the pressure on the waist and crotch of the lower spine of the user; and at the same time, the spine of the user is straightened and stabilized, is then pulled by the gravity, and becomes more natural and upright.

Based on the lever principle and the torque balance principle in the present disclosure, the designed dynamic force conversion structure embodies the following technical characteristics and functions that achieve the effects:
1) No energy is consumed. The gravity is converted for use: the gravity is converted into the embrace-like support force, so no external energy is needed.
2) It is extremely convenient. The "sitting" behavior and the embrace-like support action simultaneously appear and disappear, and there is no need to add more operations than the previous method, or the embrace-like support action is generally the same as the traditional "sitting" action. When a user needs to "sit" down, the embrace-like support force that is automatically generated at the same time when the "sitting" behavior occurs, which shares the body weight pressure with the "sitting" part and corrects the body. When the user no longer needs to "sit" down, the embrace-like support force is automatically released once "standing up" that is a reverse action of "sitting" occurs.
   That is, a major revolutionary function of reducing the pressure on the spine and correcting the spine is creatively realized without adding additional actions. It is extremely convenient and revolutionarily changes the connotation of the age-old "sitting" behavior. The harms of the traditional "sitting" behavior are basically eliminated!
3) Along the gravity direction, the spine is more natural and upright. The gravity embrace-like support and sitting device is symmetrical and balanced. When the waist and back are in an embrace-like supported state, the spine is fixed and straightened. Since the embrace-like supported part is supported upward by the friction force, the body below the embrace-like supported part is not subjected to the upper pressure, but is instead pulled up by this part. Under the action of the gravity, an extremely upright traction towards a gravity direction is generated for the entire spine, so the spine is more upright and natural.

The content described in the embodiments of this specification is merely an enumeration of the realization forms of the inventive concept, and the protection scope of the present disclosure should not be regarded as being limited to the specific forms stated in the embodiments. The protection scope of the present disclosure the equivalent technical means that can be thought by those skilled in the art according to the idea of the present disclosure.

## Claims

1. A spine-protecting and spine-correcting multi-functional health-care seat device employing gravity to achieve embrace-like support, pressure reduction, fixing, and correction, comprising a seat frame and a gravity embrace-like support and sitting device arranged on the seat frame, wherein the gravity embrace-like support and sitting device converts the gravity of a user into an embrace-like support force to the waist and back of the user, thus realizing an up support, stabilization, and correction for the waist and back of the user, relieving the pressure on the waist and hip parts, and avoiding the spine deformity.

2. The spine-protecting and spine-correcting multi-functional health-care seat device employing gravity to achieve embrace-like support, pressure reduction, fixing, and correction according to clam 1, wherein the gravity embrace-like support and sitting device comprises a sitting plate (1), sitting plate support rods (8) symmetrically arranged on two sides of a bottom of the sitting plate (1), rotating devices symmetrically arranged on two sides of the seat frame, embrace-like support levers cooperatively arranged on the rotating devices, and embrace-like support palms (2) arranged at top ends of the embrace-like support levers; one end of each sitting plate support rod (8) is hinged to a bottom of the sitting plate (1), and the other end is hinged to a bottom of each embrace-like support lever through a support rod rotating shaft (15); the sitting plate (1) may drive the sitting plate support rods (8) to move, and the sitting plate support rods (8) may drive the embrace-like support levers to move; and the embrace-like support levers may rotate on the seat frame through the rotating devices, thus realizing movement of the embrace-like support palms (2).

3. The spine-protecting and spine-correcting multi-functional health-care seat device employing gravity to achieve embrace-like support, pressure reduction, fixing, and correction according to clam 2, wherein the seat frame comprises a bottom frame and a U-shaped horizontal bracket (13) arranged on the bottom frame; the bottom frame comprises a roller base (9) and an L-shaped bracket (12) arranged on the roller base (9); the L-shaped bracket (12) is provided with an adjustable headrest (14); the U-shaped horizontal bracket (13) comprises a horizontal transverse rod and horizontal longitudinal rods arranged at two ends of the horizontal transverse rod; the L-shaped bracket (12) of the bottom frame is further provided with a U-shaped vertical support frame (10); the U-shaped vertical support frame (10) comprises a transverse rod and vertical rods arranged at two ends of the transverse rod; the vertical rods are supported at end parts of the horizontal longitudinal rods or positions close to the end parts; the rotating devices adopt rotating bearings (1101); the rotating bearings (1101) are arranged at the end parts of the horizontal longitudinal rods or positions close to the end parts; and inner rings of the rotating bearings (1101) are in tight fit with the horizontal longitudinal rods.

4. The spine-protecting and spine-correcting multi-functional health-care seat device employing gravity to achieve embrace-like support, pressure reduction, fixing, and correction according to clam 2, wherein the seat frame comprises a bottom plate (24) and support pillars (25) arranged at four corners of the bottom plate (24); tops of the support pillars (25) are provided with bearing pedestals (23); the rotating devices adopt rotating shafts (20), and two ends of the rotating shafts (20) are respectively fitted in the bearing pedestals (23); through holes are formed in the rotating shafts (20); the embrace-like support levers comprise embrace-like support connecting rods (21); the embrace-like support connecting rods(21) are arranged in the through holes in a penetrating manner and are fixedly connected with the rotating shafts (20); the roller base (9) is fixedly arranged at a bottom of the bottom plate (24); the adjustable headrest (14) is arranged on a side surface of the bottom plate (24), thus forming a chair structure; or a binding band (19) is arranged at the bottom of the bottom plate (24), thus forming a portable seat structure that can be matched with an ordinary chair.

5. The spine-protecting and spine-correcting multi-functional health-care seat device employing gravity to achieve embrace-like support, pressure reduction, fixing, and correction according to clam 2, wherein upper arms of the embrace-like support levers are connected with two connecting links (3), and the connecting links (3) and the embrace-like support levers are connected by movable joint locks, and a horizontal adjustable included angle is 45-90 degrees; an embrace-like support palm skeleton (201) and universal movable joints (202) are arranged on back surfaces of the embrace-like support palms (2); the connecting links (3) are connected with the universal movable joints (202); and front surfaces of the embrace-like support palms (2) are made of a cotton and linen material with a high elastic sponge cover friction coefficient and are matched with sunken palm type structures with small upper parts and large lower parts.

6. The spine-protecting and spine-correcting multi-functional health-care seat device employing gravity to achieve embrace-like support, pressure reduction, fixing, and correction according to clam 3, wherein each embrace-like support lever comprises an embrace-like support rod upper arm (4), an embrace-like support rod lower arm (6), and a bearing outer sleeve (11); the embrace-like support rod upper arm (4) and the embrace-like support rod lower arm (6) are welded at opposite directions on an outer wall of the bearing outer sleeve (11); the bearing outer sleeve (11) is in tight fit with an outer ring of the rotating bearing (1101); end parts of the horizontal longitudinal rods of the U-shaped horizontal bracket (13) are provided with rotating shaft lock brakes (5); the rotating shaft lock brakes (5) comprise threaded rods (503) arranged inside the horizontal longitudinal rods, transmission rods (505) oppositely arranged on the horizontal longitudinal rods in a penetrating manner, and brake handles (501) in screwing fit with the threaded rods (503); front ends of the brake handles (501) are circumferentially provided with second wedge-shaped surfaces 502); the transmission rods (505) are provided with reset springs (504); one end of each reset spring (504) is fixed on each transmission rod (505), and the other end is fixed on an inner wall of each horizontal longitudinal rod; a third wedge-shaped surface (507) is arranged at one end of each transmission rod (505) close to each threaded rod (503); a brake pad (506) is arranged at the other end of each transmission rod (505); an outer edge of each bearing outer sleeve (11) is circumferentially provided with a first wedge-shaped surface (1103); and the brake handles(501) may move on the threaded rods (503) through threaded fit and cooperate with the third wedge-shaped surfaces (507) on the transmission rods (505) through the second wedge-shaped surfaces (502), thus driving the transmission rods (505) to do relative motion, so that the brake pads (506) are fitted to the first wedge-shaped surfaces (1103) to perform a brake action.

7. The spine-protecting and spine-correcting multi-functional health-care seat device employing gravity to achieve embrace-like support, pressure reduction, fixing, and correction according to clam 3, wherein each embrace-like support lever comprises an embrace-like support rod (18) and a bearing outer sleeve (11); the bearing outer sleeve(11) is in tight fit with an outer ring of each rotating bearing (1101); the embrace-like support rod (18) is provided with one group of hooks (17); a ringlike groove (1102) is formed in the bearing outer sleeve (11); the embrace-like support rod (18) is clamped and fixed in the ringlike groove (1102) of the bearing outer sleeve (11) through the hooks; a lower end of the embrace-like support rod (18) is provided with a tension spring (7); one end of the tension spring (7) is fixedly connected with the embrace-like support rod (18), and the other end is provided with a spring force adjustment device(16); the spring force adjustment device (16) comprises a shell (1601), an upper rack (1602), a lower rack (1604), and a gear (1603); the gear (1603) is engaged with the upper rack(1602) and the lower rack (1604); the upper rack(1602) and the lower rack (1604) are arranged in the shell (1601) in a sliding fit manner; the upper rack(1602) and the lower rack (1604) are fixedly connected with the tension spring (7), respectively; the gear (1603) is connected and provided with a rotatable adjustment handle (1605); a rotating shaft of the rotatable adjustment handle (1605) is provided with a lock cylinder (1607); a lock hole (1606) is formed in a position, corresponding to the lock cylinder (1607), on the shell (1601), and the lock cylinder (1607) may cooperate with the lock hole (1606) for locking; a front end of the rotating shaft of the rotatable adjustment handle (1605) is provided with a spring (1608) used to axially reset the rotatable adjustment handle (1605); one end of the spring (1608) is fixedly connected with the rotating shaft of the rotatable adjustment handle (1605), and the other end is fixedly connected with an inner wall of the shell (1601).

8. The spine-protecting and spine-correcting multi-functional health-care seat device employing gravity to achieve embrace-like support, pressure reduction, fixing, and correction according to clam 4, wherein tail ends of the rotating shafts (20) are provided with rotating shaft lock brakes (5); the rotating shaft lock brakes (5) comprise threaded rods (503) arranged inside the rotating shafts (20), transmission rods (505) arranged at opposite positions of the rotating shafts (20) in a penetrating manner, and brake handles (501) in screwing fit with the threaded rods (503); front ends of the brake handles (501) are circumferentially provided with second wedge-shaped surfaces (502); the transmission rods (505) are provided with reset springs (504); one end of each reset spring (504) is fixed on each transmission rod (505), and the other end is fixed on an inner wall of each rotating shaft (20); a third wedge-shaped surface (507) is arranged at one end of each transmission rod (505) close to each threaded rod (503); a brake pad(506) is arranged at the other end of each transmission rod (505); an outer edge of each bearing pedestal (23) is circumferentially provided with a first wedge-shaped surface (1103); and the brake handles (501) may move on the threaded rods (503) through threaded fit and cooperate with the third wedge-shaped surfaces (507) on the transmission rods (505) through the second wedge-shaped surfaces (502), thus driving the transmission rods (505) to do relative motion, so that the brake pads (506) are fitted to the first wedge-shaped surfaces(1103) to perform a brake action.

9. The spine-protecting and spine-correcting multi-functional health-care seat device employing gravity to achieve embrace-like support, pressure reduction, fixing, and correction according to clam 4, wherein pin holes located on side surfaces of the through holes are formed in the rotating shafts (20), and the pin holes communicate with the through holes; one group of spring pins (22) are arranged on the embrace-like support connecting rods (21); the embrace-like support connecting rods (21) are arranged on the rotating shafts (20) through the through holes in a penetrating manner and cooperate with the pin holes through spring pins (22) to realize upper-lower ratio adjustment of the embrace-like support connecting rods (21) on the rotating shafts (20); a lower end of each embrace-like support connecting rod (21) is provided with a tension spring (7); one end of the tension spring (7) is fixedly connected with the embrace-like support connecting rod (21), and the other end is provided with a spring force adjustment device(16); the spring force adjustment device (16) comprises a shell (1601), an upper rack (1602), a lower rack (1604), and a gear (1603); the gear (1603) is engaged with the upper rack(1602) and the lower rack (1604); the upper rack(1602) and the lower rack (1604) are arranged in the shell (1601) in a sliding fit manner; the upper rack(1602) and the lower rack (1604) are fixedly connected with the tension spring (7), respectively; the gear (1603) is connected and provided with a rotatable adjustment handle (1605); a rotating shaft of the rotatable adjustment handle (1605) is provided with a lock cylinder (1607); a lock hole (1606) is formed in a position, corresponding to the lock cylinder (1607), on the shell (1601), and the lock cylinder (1607) may cooperate with the lock hole (1606) for locking; a front end of the rotating shaft of the rotatable adjustment handle (1605) is provided with a spring (1608) used to axially reset the rotatable adjustment handle (1605); one end of the spring (1608) is fixedly connected with the rotating shaft of the rotatable adjustment handle (1605), and the other end is fixedly connected with an inner wall of the shell (1601).

10. The spine-protecting and spine-correcting multi-functional health-care seat device employing gravity to achieve embrace-like support, pressure reduction, fixing, and correction according to clam 6 or 8, wherein tension springs (7) are arranged in gaps between the embrace-like support levers and the sitting plate support rods (8); one end of each tension spring (7) is fixedly connected with each embrace-like support lever, and the other end is fixedly connected with the sitting plate support rod (8).
